# EUROPEAN PATENT APPLICATION

(11) **EP 4 122 522 A1**
(43) Date of publication of application: **25.01.2023**
(21) Application number: 20925096.8
(22) Date of filing: 19.03.2020
(51) Int. Cl.: A61M 25/14

(54) **CATHETER**

(71) Applicant: Asahi Intecc Co., Ltd., Seto-shi, Aichi 489-0071 (JP)
(72) Inventor: ENDO Shota, Seto-shi, Aichi 489-0071 (JP)
(74) Representative: Winter, Brandl - Partnerschaft mbB
(86) International application number: PCT/JP2020/012236
(87) International publication number: WO 2021/186664

(57) **Abstract**

A shaft of a catheter is formed with a first lumen that communicates from the proximal end to the distal end of the shaft, and a second lumen that is positioned on the outer periphery side of the first lumen, communicates from the proximal end side to the distal end side of the shaft, and has a smaller opening area than the opening area of the first lumen in a transverse section of the shaft. The dimension in the first direction in the distal end portion of the shaft where the first lumen and the second lumen are aligned is less than or equal to the dimension in the first direction in the proximal end portion that is closer to the proximal end side than the distal end portion of the shaft.

## Description

### TECHNICAL FIELD

The technique disclosed herein relates to a catheter.

### BACKGROUND ART

Several catheter-based methods have been widely employed to treat or examine a constricted part, an occluded part, an abnormal blood vessel, or the like in a blood vessel or the like (hereinafter referred to as "lesion"). Some catheters are multi-lumen type catheters with multiple lumens (see Patent Literatures 1 and 2 below). Specifically, the multi-lumen type catheter has a shaft and a balloon joined to the distal end portion of the shaft, and the shaft is formed with a main lumen and an expansion lumen. The main lumen is a lumen through which a device such as a guide wire, for example, is inserted. The expansion lumen is positioned on the outer periphery side of the main lumen and is a lumen through which a fluid that inflates the balloon is circulated. In a transverse section of the shaft, the opening area of the expansion lumen is smaller than that of the main lumen.

### CITATION LIST

### Patent Literature

Patent Literature 1: JP 2012-143377 A
Patent Literature 2: WO 2015/013612

### SUMMARY OF INVENTION

### TECHNICAL PROBLEM

In the conventional multi-lumen type catheter described above, the opening width of the expansion lumen in the first direction where the main lumen and the expansion lumen are aligned is the same over the entire length of the shaft. Therefore, in the distal end portion of the shaft, the main lumen is positioned at a large eccentricity with respect to the center of the shaft due to the presence of the expansion lumen, and the radical thickness on the main lumen side in the distal end portion of the shaft becomes thin. As a result, the strength of the distal end portion of the shaft is low, and there is a possibility that it will be easily damaged by bending or the like.

Such issue is not limited to balloon catheters, but are common to multi-lumen type catheters.

Disclosed herein is a technique that provides a solution to the issue described above.

### SOLUTION TO PROBLEM

The technique disclosed herein can be achieved as the following aspects, for example.
(1) A catheter disclosed herein is a catheter including a shaft. The shaft is formed with a first lumen that communicates from a proximal end to a distal end of the shaft and a second lumen that is positioned on an outer periphery side of the first lumen, communicates from a proximal end side to a distal end side of the shaft, and has a smaller opening area than an opening area of the first lumen in a transverse section of the shaft. A dimension in a first direction in a distal end portion of the shaft where the first lumen and the second lumen are aligned is less than or equal to a dimension in the first direction in a proximal end portion that is closer to the proximal side than the distal end portion of the shaft. An opening width in the first direction of the second lumen in a transverse section of the distal end portion of the shaft is smaller than an opening width in the first direction of the second lumen in a transverse section of the proximal end portion of the shaft. In the distal end portion of the shaft, a radical thickness on the first lumen side in the first direction is thicker than a radical thickness on the second lumen side in the first direction. According to this catheter, it is possible to prevent the dimension of the distal end portion of the shaft from becoming larger than the dimension of the proximal end portion and the difference in the opening width in the first direction of the first lumen, and to secure the radical thickness on the first lumen side in the distal end portion of the shaft and improve the strength.
(2) In the above catheter, an opening width in a second direction perpendicular to the first direction of the second lumen in a transverse section of the distal end portion of the shaft may be larger than an opening width in the second direction of the second lumen in a transverse section of the proximal end portion of the shaft. According to this catheter, compared to the configuration in which the opening width in the second direction perpendicular to the first direction of the second lumen in a transverse section of the distal end portion of the shaft is less than or equal to the opening width in the second direction of the second lumen in a transverse section of the proximal end portion of the shaft, it is possible to suppress an increase in the flow channel resistance of the second lumen due to a decrease in the opening area of the second lumen.
(3) In the above catheter, an opening area of the second lumen in a transverse section of the distal end portion of the shaft may be substantially same as an opening area of the second lumen in a transverse section of the proximal end portion of the shaft. According to this catheter, compared to the configuration in which the opening area of the second lumen differs between the distal end portion and the proximal end portion of the shaft, it is possible to more effectively suppress an increase in the flow channel resistance of the second lumen due to a decrease in the opening area of the second lumen.
(4) In the above catheter, a balloon that is joined to the distal end portion of the shaft and communicates with the second lumen may be further included. This catheter is particularly useful because it improves the strength of the distal end portion of the shaft, that is joined to the balloon.

### BRIEF DESCRIPTION OF DRAWINGS

FIG. 1 is an explanatory diagram (longitudinal sectional view) schematically illustrating a configuration of a balloon catheter 100 in a first embodiment.
FIG. 2 is an explanatory diagram (transverse sectional view) schematically illustrating a distal end portion 12 of a shaft 10 in the first embodiment.
FIG. 3 is an explanatory diagram (transverse sectional view) schematically illustrating a proximal end portion 14 of the shaft 10 in the first embodiment.
FIG. 4 is an explanatory diagram (transverse sectional view) schematically illustrating a distal end portion 12 of a shaft 10a of a balloon catheter 100a in a second embodiment.

### EMBODIMENTS OF THE INVENTION

### A. First Embodiment:

### A-1. Basic Configuration of Balloon Catheter 100:

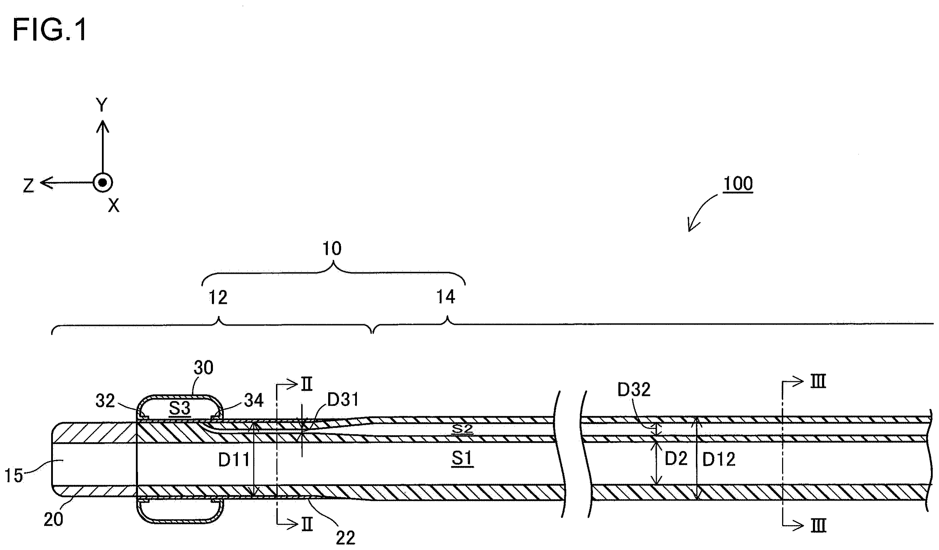

FIG. 1 is an explanatory diagram schematically illustrating a configuration of a balloon catheter 100 in a first embodiment. FIG. 1 illustrates a configuration of a longitudinal section of the balloon catheter 100 (YZ section: a sectional view cut along a plane including a Y-axis and a Z-axis described in FIG. 1). However, in FIG. 1, a portion of a shaft 10 of the balloon catheter 100 is omitted. The balloon catheter 100 is an example of the catheter in the claims.

In FIG. 1, the positive side of the Z-axis (the side of a distal tip 20 of the balloon catheter 100) is the distal end side (far side) to be inserted into the body, and the negative side of the Z-axis (the side opposite to the distal tip 20 of the balloon catheter 100) is the proximal end side (near side) to be manipulated by a technician such as a doctor. FIG. 1 illustrates a state where the balloon catheter 100 is in an entirely straight form parallel to the Z-axis direction, but the balloon catheter 100 has enough flexibility to be bent. FIG. 1 also illustrates a balloon 30 described below in its expanded state.

The balloon catheter 100 is a medical device inserted into a blood vessel or the like to push and expand a lesion (constricted part or occluded part) in a blood vessel or the like, or to occlude a blood vessel to temporarily block blood flow. The balloon catheter 100 includes a shaft 10 and a balloon 30.

As illustrated in FIG. 1, the shaft 10 has a distal end portion 12 and a proximal end portion 14. The distal end portion 12 is a portion including the distal end of the shaft 10, and is a portion in which an outer diameter D11 is smaller than an outer diameter D12 of the proximal end portion 14 and thus the flexibility is relatively high. Specifically, the distal end portion 12 has a same diameter portion and a tapered portion. The same diameter portion is a portion which includes the distal end of the shaft 10 and in which the outer diameter D11 is substantially the same over the entire length. The tapered portion is positioned between the same diameter portion and the proximal end portion 14, and the outer diameter of the tapered portion increases as approaching the proximal end portion 14. The outer diameter D11 of the distal end portion 12 is an example of the dimension in the first direction of the distal end portion in the claims, and the outer diameter D12 of the proximal end portion 14 is an example of the dimension in the first direction of the proximal end portion in the claims. In this specification, the description that A and B are substantially same means that the error between A and B is less than or equal to 5% of A or B.

The proximal end portion 14 of the shaft 10 is a portion including the proximal end of the shaft 10, and is a portion in which the outer diameter D12 is larger than the outer diameter D 11 of the distal end portion 12 (the above same diameter portion) and thus the stiffness is relatively high. The outer diameter D12 of the proximal end portion 14 is substantially the same over the entire length of the proximal end portion 14. The distal end portion 12 is, for example, the portion within 15 cm from the distal end of the shaft 10. At the proximal end of the proximal end portion 14, a connector (not illustrated) for introducing a device, a fluid, or the like into each lumen S1 and S2) is attached. The configuration of the shaft 10 will be described in detail below.

The shaft 10 is a tubular (e.g., cylindrical) member with openings at the distal end and proximal end. As used herein, "tubular (cylindrical)" is not limited to a completely tubular (cylindrical) shape, but may also be an overall substantially tubular shape (a substantially cylindrical shape such as a slightly conical shape or a partially uneven shape). Inside the shaft 10, a main lumen S1 through which a linear device (not illustrated) such as a guide wire or a dilator is inserted and an expansion lumen S2 through which an expansion fluid for expanding the balloon 30 flows are formed. The fluid can be a gas or a liquid, and a gas such as helium gas, CO₂ gas, and O₂ gas, or a liquid such as physiological saline or contrast medium can be given as an example. In other words, the balloon catheter 100 is a so-called two-lumen type catheter including the main lumen S1 and the expansion lumen S2. The main lumen S 1 is an example of the first lumen in the claims, and the expansion lumen S2 is an example of the second lumen in the claims. The specific configuration of each lumen S1 and S2 will be described below.

The shaft 10 is preferably made of a material that can be heat-sealed and has a certain degree of flexibility. Examples of the material for forming the shaft 10 include a thermoplastic resin, more specifically polyethylene, polypropylene, polybutene, ethylene-propylene copolymer, ethylene-vinyl acetate copolymer, ionomer, or a polyolefin such as a mixture of two or more of these, polyvinyl chloride resin, polyamide, polyamide elastomer, polyester, polyester elastomer, thermoplastic polyurethane, and the like.

The distal end of the shaft 10 is provided with a distal tip 20. The distal tip 20 is a cylindrical member with openings at the distal end and the rear end. The distal tip 20 has a tapered outer shape with a gradually decreasing outer diameter toward the distal end as well as a port 15 on its distal end side. A device inserted in the main lumen S1 is led out through the port 15. The distal tip 20 is formed, for example, of a resin or a metal.

As illustrated in FIG. 1, part or all of the outer peripheral surface of the distal end portion 12 of the shaft 10 has a coating layer 22 formed by a coating agent. The coating layer 22 is provided to reduce the frictional resistance between the surface of the distal end portion 12 and the inner wall of the blood vessel when the distal end portion 12 is inserted into the blood vessel, and to ensure slidability. Therefore, the coating layer 22 should be formed of a material with low frictional resistance (such as hydrophilic resin). For example, it is desirable to coat with polyvinyl alcohol, polyvinylpyrrolidone, polyethylene glycol, polyacrylamide, polyacrylic acid, sodium polyacrylate, poly (2-hydroxyethyl methacrylate), maleic anhydride-based copolymer, ethylene vinyl alcohol copolymer, 2-methacryloyloxyethyl phosphorylcholine or a copolymer thereof, (2-hydroxyethyl methacrylate)-styrene block copolymer, various synthetic polypeptides, collagen, hyaluronic acid, cellulosic polymer, and mixtures of these.

The balloon 30 is an expandable section that can expand and contract as a fluid is supplied and expelled. The balloon 30 covers the outer periphery of the distal end portion 12 of the shaft 10. A distal end 32 and a rear end 34 of the balloon 30 are respectively joined to the outer peripheral surface of the distal end portion 12 of the shaft 10, for example, by welding. The distal end of the distal tip 20 is open on the distal end side of the distal end 32 of the balloon 30. In the contracted state, the balloon 30 is folded so as to adhere to the outer peripheral surface of the shaft 10 (not illustrated). The length of the balloon 30 in the Z-axis direction is approximately 2 cm, for example.

The balloon 30 is preferably made of a material having a certain degree of flexibility, and more preferably made of a material thinner than the shaft 10 and having flexibility. Examples of the material for forming the balloon 30 include polyethylene, polypropylene, polybutene, ethylene-propylene copolymer, ethylene-vinyl acetate copolymer, ionomer, or a polyolefin such as a mixture of two or more of these, soft polyvinyl chloride resin, thermoplastic resin such polyamide, polyamide elastomer, polyester, polyester elastomer, polyurethane, and fluororesin, silicone rubber, latex rubber, and the like.

### A-2. Detailed Configuration of Each Lumen S1, S2:

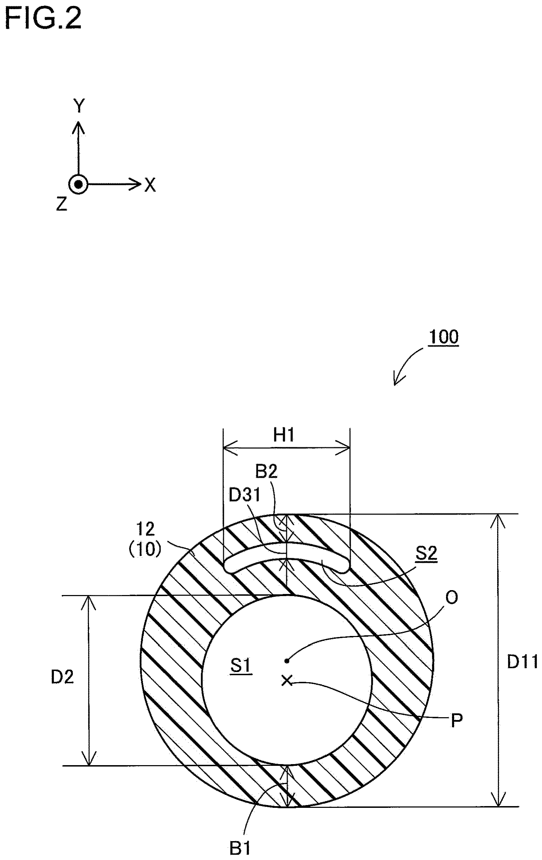

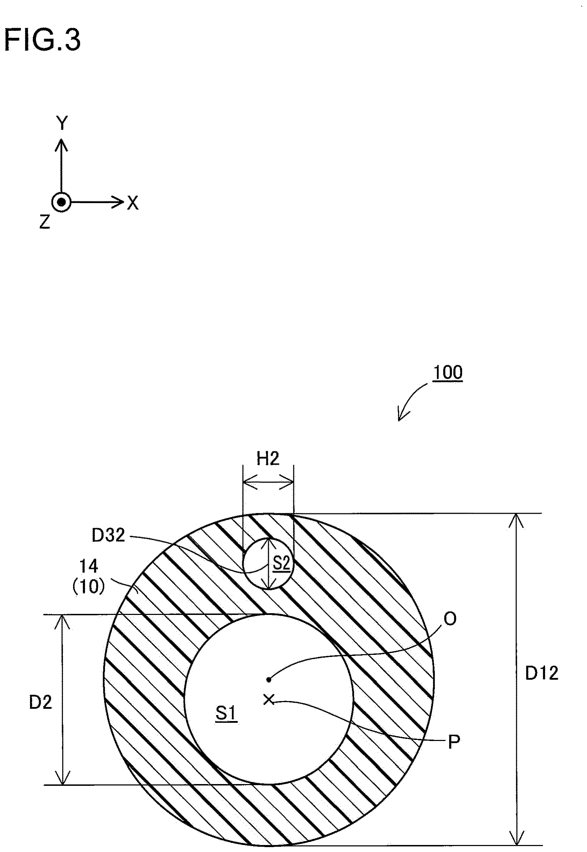

Next, the detailed configuration of each lumen S1 and S2 formed in the shaft 10 will be described. FIG. 2 is an explanatory diagram schematically illustrating the distal end portion 12 of the shaft 10. FIG. 2 illustrates a configuration of a transverse section of the shaft 10 at the position II-II of FIG. 1 (XY section: a sectional view cut along a plane including an X-axis and a Y-axis described in FIG. 2). FIG. 3 is an explanatory diagram schematically illustrating the proximal end portion 14 of the shaft 10. FIG. 3 illustrates a configuration of a transverse section of the shaft 10 at the position III-III of FIG. 1. Hereinafter, in the transverse section of the shaft 10, the direction (Y-axis direction in each figure) along the straight line connecting the center (area center of gravity) of the main lumen S1 and the center (area center of gravity) of the expansion lumen S2 is defined as a "lumen alignment direction Y". The lumen alignment direction Y is an example of the first direction in the claims.

As illustrated in FIG. 1, the main lumen S1 communicates from the proximal end to the distal end of the shaft 10. In the transverse section of the shaft 10, a center O of the shaft 10 is positioned within the contour line of the main lumen S1. However, as illustrated in FIGs. 2 and 3, the main lumen S1 is arranged at a position eccentric with respect to the center O of the shaft 10 due to the presence of the expansion lumen S2. Specifically, in the lumen alignment direction Y, the position of a center P of the main lumen S1 is deviated from the position of the center O of the shaft 10 on the side opposite to the expansion lumen S2 (Y-axis negative direction side). The opening shape of the main lumen S1 in the transverse section of the shaft 10 is substantially circular. The opening area of the main lumen S1 in the transverse section of the shaft 10 (hereinafter also simply referred to as the "opening area of the main lumen S1") is substantially the same over the entire length of the shaft 10. As mentioned above, since a linear device is inserted through the main lumen S1, the opening area of the main lumen S1 is preferably substantially the same over the entire length of the shaft 10.

As illustrated in FIG. 1, the expansion lumen S2 extends from the proximal end to the distal end portion 12 of the shaft 10. The distal end side of the expansion lumen S2 is curved laterally (Y-axis positive direction side in FIG. 1) with respect to the axial direction of the shaft 10 (Z-axis direction in each figure of the length direction of the shaft 10), opens in the outer peripheral surface of the distal end portion 12, and communicates with an internal space S3 of the balloon 30. As illustrated in FIGs. 2 and 3, the expansion lumen S2 is positioned on the outer periphery side of the main lumen S1. In the transverse section of the shaft 10, the center O of the shaft 10 is positioned outside the contour line of the expansion lumen S2. The opening area of the expansion lumen S2 in the transverse section of the shaft 10 (hereinafter also simply referred to as "opening area of the expansion lumen S2") is smaller than the opening area of the main lumen S1. Further, second opening widths D31 and D32 in the lumen alignment direction Y of the expansion lumen S2 in the transverse section of the shaft 10 (see FIG. 2) are smaller than a first opening width D2 in the lumen alignment direction Y of the expansion lumen S2 in the transverse section of the proximal end portion 14 of the shaft 10.

The second opening width D31 (see FIG. 2) of the expansion lumen S2 in the distal end portion 12 of the shaft 10 is smaller than the second opening width D32 (see FIG. 3) of the expansion lumen S2 in the proximal end portion 14. Further, as illustrated in FIG. 2, in the distal end portion 12, a radical thickness B1 on the first lumen side (opposite to the expansion lumen S2) in the lumen alignment direction Y is thicker than a radical thickness B2 on the expansion lumen S2 side in the lumen alignment direction Y

Furthermore, a third opening width H1 (see FIG. 2) in the width direction (X-axis direction in each figure) perpendicular to the lumen alignment direction Y of the expansion lumen S2 in the transverse section of the distal end portion 12 is larger than a third opening width H2 (see FIG. 3) in a width direction X of the expansion lumen S2 in the transverse section of the proximal end portion 14. The opening area of the expansion lumen S2 in the transverse section of the distal end portion 12 and the opening area of the expansion lumen S2 in the transverse section of the proximal end portion 14 are substantially the same. The opening shape (shape of the contour line) of the expansion lumen S2 in the transverse section of the distal end portion 12 is a shape in which the opening shape of the expansion lumen S2 in the transverse section of the proximal end portion 14 is flattened in the lumen alignment direction Y. Specifically, the opening shape of the expansion lumen S2 in the proximal end portion 14 is substantially circular (see FIG. 3), and the opening shape of the expansion lumen S2 in the distal end portion 12 is elliptical (see FIG. 2).

The center-to-center distance between the center P of the main lumen S1 and the center O of the shaft 10 in the transverse section of the distal end portion 12 is shorter than the same center-to-center distance in the transverse section of the proximal end portion 14. In other words, the flat shape of the opening of the expansion lumen S2 in the distal end portion 12 reduces the eccentricity width of the main lumen S1 with respect to the center O of the shaft 10. As a result, the transverse sectional shape of the distal end portion 12 is close to a perfect circle, and the distribution of the resin is close to uniform.

The expansion lumen S2 described above can be formed, for example, as follows. In a resin-formed body having holes having substantially the same diameter over the entire length, a core material having a smaller outer diameter than the hole in the proximal end portion is inserted into the hole in the distal end portion and heat-treated. This creates a substantially circular hole in the proximal end portion and a flattened hole in the distal end portion.

### A-3. Effect of the Embodiment:

As described above, in the balloon catheter 100 of the present embodiment, the outer diameter D11 of the distal end portion 12 of the shaft 10 is smaller than the outer diameter D12 of the proximal end portion 14 (see FIG. 1). The second opening width D31 (see FIG. 2) of the distal end portion 12 of the shaft 10 is smaller than the second opening width D32 (see FIG. 3) in the proximal end portion 14. As illustrated in FIG. 2, in the distal end portion 12, the radical thickness B1 on the first lumen side in the lumen alignment direction Y is thicker than the radical thickness B2 on the expansion lumen S2 side in the lumen alignment direction Y As a result, it is possible to prevent the dimension of the distal end portion 12 of the shaft 10 from becoming larger than the dimension of the proximal end portion 14 and variation of the first opening width D2 of the main lumen S1, and to secure the radical thickness B1 on the main lumen S1 side in the distal end portion 12 and improve the strength of the distal end portion 12.

For example, in the configuration in which the second opening width D31 of the expansion lumen S2 in the distal end portion 12 of the shaft 10 is the same as the second opening width D32 of the expansion lumen S2 in the proximal end portion 14, since the transverse sectional shape of the distal end portion 12 has a protruding shape in the vicinity portion of the expansion lumen S2, the distal end portion 12 may be difficult to bend in a particular direction. In contrast, in the above embodiment, the transverse sectional shape of the distal end portion 12 can be suppressed to have a protruding shape in the vicinity portion of the expansion lumen S2. Specifically, the transverse sectional shape of the distal end portion 12 is substantially circular. This prevents the distal end portion 12 from becoming difficult to bend in a particular direction.

In the present embodiment, the third opening width H1 (see FIG. 2) of the expansion lumen S2 in the distal end portion 12 is larger than the third opening width H2 (see FIG. 3) of the expansion lumen S2 in the proximal end portion 14. As a result, compared to the configuration in which the third opening width H1 of the expansion lumen S2 in the distal end portion 12 is less than or equal to the third opening width H2 of the expansion lumen S2 in the proximal end portion 14, it is possible to suppress an increase in the flow channel resistance of the expansion lumen S2 due to a decrease in the opening area of the expansion lumen S2.

In the present embodiment, the opening area of the expansion lumen S2 in the transverse section of the distal end portion 12 and the opening area of the expansion lumen S2 in the transverse section of the proximal end portion 14 are substantially the same. Compared to the configuration in which the opening area of the expansion lumen S2 differs between the distal end portion 12 and the proximal end portion 14 of the shaft 10, it is possible to more effectively suppress an increase in the flow channel resistance of the expansion lumen S2 due to a decrease in the opening area of the expansion lumen S2. Further, since the expansion lumen S2 in the distal end portion 12 and the expansion lumen S2 in the proximal end portion 14 are connected at a tapered portion whose width is continuously narrowed toward the distal end of 10, compared to the configuration in which the expansion lumen S2 in the distal end portion 12 and the expansion lumen S2 in the proximal end portion 14 are connected via a stepped surface, it is possible to more effectively suppress an increase in the flow channel resistance of the expansion lumen S2.

According to the above embodiment, due to the radical thickness B1 on the main lumen S1 side of the distal end portion 12 of the shaft 10, a sufficient amount of resin is used for welding with the balloon 30, and thus the joint strength and adhesion between the distal end portion 12 and the balloon 30 are improved.

### B. Second Embodiment:

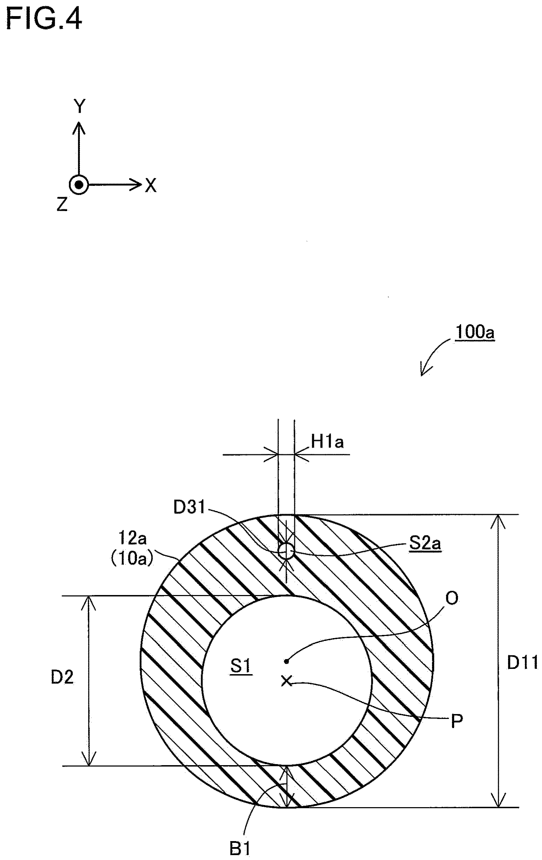

FIG. 4 is an explanatory diagram schematically illustrating a distal end portion 12 of a shaft 10a of a balloon catheter 100a in a second embodiment. FIG. 4 illustrates a configuration of a transverse section of the shaft 10a. Hereinafter, in the configurations of the balloon catheter 100a of the second embodiment, the same configurations as those of the balloon catheter 100 of the first embodiment described above will be appropriately omitted by adding the same reference numerals.

As illustrated in FIG. 4, in the balloon catheter 100a of the second embodiment, a third opening width H1a of an expansion lumen S2a in the transverse section of a distal end portion 12a of a shaft 10a is smaller than a third opening width H2 of an expansion lumen S2 in the proximal end portion 14 (see FIG. 3). The third opening width H1a of the expansion lumen S2a in the distal end portion 12a is substantially the same as the second opening width D31 in the lumen alignment direction Y of the expansion lumen S2a. In other words, the shape of the expansion lumen S2a in the transverse section of the distal end portion 12a is substantially circular.

According to this second embodiment, the shape of the expansion lumen S2a is simpler than that of the first embodiment above, and thus it is easier to manufacture the shaft 10a. The opening area of the expansion lumen S2 in the transverse section of the proximal end portion 14 of the shaft 10a is relatively large. Therefore, compared to the configuration in which the opening area of the expansion lumen S2 in the transverse section of the proximal end portion 14 of the shaft 10a is the same as the opening area of the expansion lumen S2 in the transverse section of the distal end portion 12a, a fluid can be supplied and discharged to the balloon 30 at an early stage. Thus, the responsiveness of expansion and contraction of the balloon 30 is improved.

### C. Modifications:

The technique disclosed herein is not limited to the embodiments described above, and can be modified to a variety of aspects within the range not departing from its spirits; for example, the following modifications are also available.

The configurations of the balloon catheters 100 and 100a in the above embodiments are just an example, and can be modified variously. For example, in the above embodiments, the balloon 30 may not be included. In this configuration, the expansion lumen S2 functions as a lumen to which a drug such as an embolic substance to be injected into an abnormal blood vessel is supplied. Further, the expansion lumen S2 may be connected to the distal end of the shaft 10. Further, in the above embodiments, a plurality of expansion lumens S2 may be formed in the shaft 10 (however, for example, an embodiment in which the expansion lumen S2 is present on both sides of the main lumen S1 in the lumen alignment direction Y is excluded). Further, in the above embodiments, the expansion lumen S2 in the distal end portion 12 and the expansion lumen S2 in the proximal end portion 14 may be connected via a stepped surface. Furthermore, in the above embodiments, the coating layer 22 may not be included.

In the above embodiments, an example in which the disclosed embodiments are applied to an over-the-wire type configuration in which the main lumen S1 communicates from the proximal end to the distal end of the shaft 10 has been described. However, the disclosed embodiments can also be applied to a rapid exchange type configuration. In the case of the rapid exchange type configuration, the main lumen S1 is required to have the same configuration as that of the above embodiments in the range illustrated in FIG. 1 of the catheters.

In the above embodiments, the outer diameter of the shaft 10 may be substantially the same over the entire length. Further, the transverse sectional shape of the shaft 10 is not limited to a circular shape, but may also be polygonal or other shapes. Further, a braided body, a coil body, or the like may be embedded in the distal end portion 12 of the shaft 10. By applying the disclosed embodiments to such a configuration, the radical thickness can be secured, especially on the main lumen S1 side of the distal end portion 12. Therefore, it is possible to suppress the deterioration of the joint performance with the balloon 30 and the coating layer 22 due to the braided body or the like embedded in the distal end portion 12 being exposed to the outside.

The material of each member of the embodiments described above is merely an example, and can be modified in various ways.

### DESCRIPTION OF REFERENCE NUMERALS

10, 10a shaft
12, 12a distal end portion
14 proximal end portion
15 port
20 distal tip
22 coating layer
30 balloon
32 distal end
34 rear end
100, 100a balloon catheter
B1, B2 radical thickness
D11, D12 outer diameter
D2, D31, D32 opening width
H1, H1a, H2 opening width
O, P center
S1 main lumen
S2, S2a expansion lumen
S3 internal space
X width direction
Y lumen alignment direction

## Claims

1. A catheter comprising a shaft,
wherein the shaft is formed with a first lumen that communicates from a proximal end to a distal end of the shaft and a second lumen that is positioned on an outer periphery side of the first lumen, communicates from a proximal end side to a distal end side of the shaft, and has a smaller opening area than an opening area of the first lumen in a transverse section of the shaft;
wherein a dimension in a first direction in a distal end portion of the shaft where the first lumen and the second lumen are aligned is less than or equal to a dimension in the first direction in a proximal end portion that is closer to the proximal side than the distal end portion of the shaft,
wherein an opening width in the first direction of the second lumen in a transverse section of the distal end portion of the shaft is smaller than an opening width in the first direction of the second lumen in a transverse section of the proximal end portion of the shaft, and
wherein in the distal end portion of the shaft, a radical thickness on the first lumen side in the first direction is thicker than a radical thickness on the second lumen side in the first direction.

2. The catheter according to Claim 1, wherein an opening width in a second direction perpendicular to the first direction of the second lumen in a transverse section of the distal end portion of the shaft is larger than an opening width in the second direction of the second lumen in a transverse section of the proximal end portion of the shaft.

3. The catheter according to Claim 2, wherein an opening area of the second lumen in a transverse section of the distal end portion of the shaft is substantially same as an opening area of the second lumen in a transverse section of the proximal end portion of the shaft.

4. The catheter according to any one of Claims 1 to 3, further comprising a balloon that is joined to the distal end portion of the shaft and communicates with the second lumen.
